# EUROPEAN PATENT APPLICATION

(11) **EP 4 043 557 A1**
(43) Date of publication of application: **17.08.2022**
(21) Application number: 21748220.7
(22) Date of filing: 26.01.2021
(51) Int. Cl.: C12N 7/04, A61L 2/12, A61L 27/36, C12N 5/071

(54) **VIRAL INACTIVATION METHOD, METHOD FOR PRODUCING DRIED AMNION, AND DRIED AMNION**

(30) Priority: 29.01.2020 JP 2020012404
(71) Applicant: SAKURA SEIKI CO., LTD., Chikuma-shi Nagano 387-0015 (JP); Yoshida, Toshiko, Toyama-shi, Toyama, 931-8303 (JP); Hayashi, Kyouko, Imizu-shi Toyama 939-0341 (JP)
(72) Inventor: ARAKAWA Masahiko, Chikuma-shi, Nagano 387-0015 (JP); OKABE Motonori, Imizu-shi, Toyama 939-0364 (JP); YOSHIDA Toshiko, Toyama-shi, Toyama 931-8306 (JP); HAYASHI Kyouko, Imizu-shi, Toyama 939-0341 (JP)
(74) Representative: Mewburn Ellis LLP
(86) International application number: PCT/JP2021/002520
(87) International publication number: WO 2021/153524

(57) **Abstract**

An object is to develop technology for viral inactivation. As means for resolution, viruses are inactivated by irradiating various articles with microwaves.

## Description

### Technical Field

The present invention relates to a viral inactivation method, a method for producing dried amnion and a dried amnion.

### Background Art

A capsid is a protein shell enclosing the genome of a virus and is denatured by heat treatment at 56°C for 30 minutes or at 100°C for five minutes, and thus the virus is inactivated. Examples of the applications thereof include "liquid heat treatment", in which viruses are inactivated by heating a plasma-derived product in the solution state at 60°C for 10 hours, and "dry heat treatment" for blood coagulation factor products and the like, in which a drug solution is freeze-dried in a vacuum and then heated under the conditions of "at 65°C for 96 hours".

Microwaves are used as heating means for inactivating viruses, and it is known that microwave heating inactivates viruses. Moreover, an attempt has been made to inactivate an influenza virus adhered to a mask with a microwave oven using microwave heating (PTL 1).

For drying vegetables, fruits and the like, "vacuum drying (reduced pressure drying)", which is a method in which the object to be dried (a vegetable, a fruit or the like) is placed in a sealed container and in which the pressure is reduced and the air is exhausted with a vacuum pump to artificially increase the difference in the partial pressure of water vapor, is known. In vacuum drying, to prevent the temperature of the material to be dried from decreasing with the moisture vaporization, a method in which the temperature is increased before the material is placed in the container, a method in which the entire container is heated to heat the material to be dried with radiant heat, a method in which the material to be dried is placed on a heater plate in the container and heated with heat conduction and other methods are generally used. Moreover, a production method and a production apparatus for a dried product with extremely low oxidization by heating using microwaves and far-infrared rays in combination in a vacuum, in which the boiling temperature is low, to shorten the drying period are known (PTL 2).

### Citation List

### Patent Literature

PTL 1: JP-A-2011-15822
PTL 2: JP-A-2000-220963

### Summary of Invention

### Technical Problem

In production steps of a material for regenerative medicine such as human or animal cells or tissues, it is considered to be often difficult to include a step of inactivating/removing infectious substances such as viruses. When viral inactivation/removal treatment is possible, however, it is required to carry out the treatment in principle.

Moreover, viral inactivation is generally required for foods and the like in addition to materials for regenerative medicine.

### Solution to Problem

An amnion is a membrane in which the fetus grows and is discarded after birth but has long been used as a material for covering a wound due to its antiinflammatory effect. Recently, a cryopreserved amnion is used as a material for regenerative medicine in the ophthalmology field. Practical application of a dried amnion produced by drying an amnion using far-infrared rays and microwaves in combination under reduced pressure (a hyper dry amnion) is under examination.

The present inventors have found that microwave irradiation under reduced pressure during the production steps of a hyper dry amnion is a useful method as viral inactivation means for materials for regenerative medicine and have also found that microwave irradiation under reduced pressure is useful also for general foods and the like in addition to materials for regenerative medicine.

### Advantageous Effects of Invention

By irradiating various articles with microwaves under reduced pressure, viruses can be inactivated.

Moreover, in the production process of a hyper dry amnion, the amnion can be dried, and the viruses can be inactivated.

### Brief Description of Drawings

Fig. 1 is a graph showing inactivation of poliovirus.
Fig. 2 is a graph showing inactivation of an influenza virus.
Fig. 3 is a graph showing inactivation of herpes virus type 1.
Fig. 4 is a graph showing inactivation of herpes virus type 1.
Fig. 5 is a graph showing inactivation of poliovirus.
Fig. 6 is a schematic view of a viral inactivation apparatus.

### Description of Embodiments

The invention is explained in detail below.

In the invention, the pressure reduction is to reduce the pressure in the container to a slightly reduced pressure of 0.1 Kp to 6.0 Kp, preferably 0.3 Kpa to 5.5 Kpa, of the low vacuum in the category of vacuum defined by JIS (Japanese Industrial Standards) in Japan (degree of vacuum of 100 pascals (0.1 Kp) or more). Moreover, the pressure reduction is preferably repeated multiple times in the range of 0.1 Kp to 6.0 Kp and is further preferably repeated multiple times in the range of 0.3 Kpa to 5.5 Kpa.

The microwaves in the invention are radio waves of a frequency of 300 MHz to 300 GHz (a wavelength of 1 m to 1 mm). The microwaves used in the invention are not particularly limited as long as the microwaves are radio waves of a frequency assigned by the ITU (International Telecommunication Union) for industrial, scientific and medical purposes (an ISM frequency), but microwaves of the 2450 MHz band, which are used for a microwave oven and the like, are preferable.

An example of the oscillating pipe for generating the microwaves is a small light-weight permanent magnet-bearing magnetron, and the output thereof is 300 W to 10 kW. A preferable output is 1 kW to 3 kW.

The output of the microwaves during the microwave irradiation should be 100 W or more and is preferably 100 W to 300 W. During the microwave irradiation, the object (a material for regenerative medicine or the like) may be separately irradiated with far-infrared rays.

The virus inactivated in the invention is not particularly limited but is a virus having an envelope such as herpes simplex viruses, influenza viruses and human immunodeficiency viruses or a virus without any envelope such as noroviruses, rotaviruses and adenoviruses.

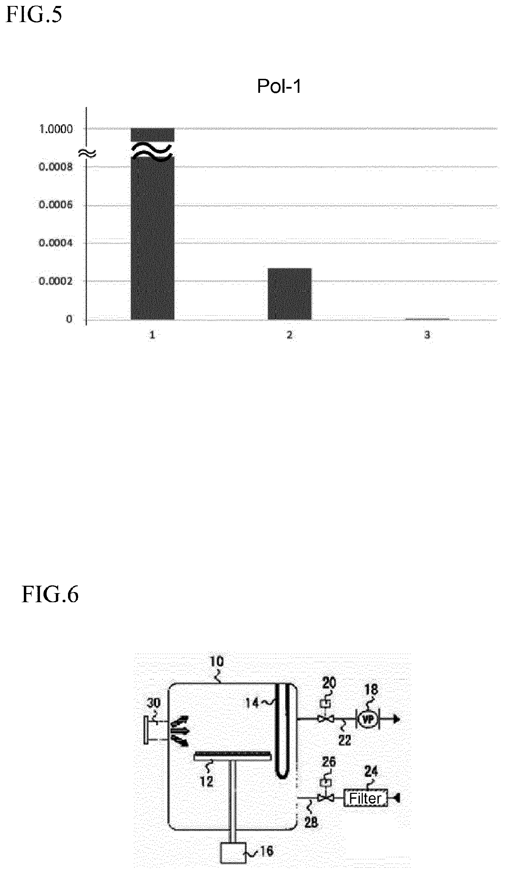

An example of the apparatus for carrying out the method of the invention is an apparatus having the structure which is schematically illustrated in Fig. 6. The apparatus is explained below.

In the apparatus, a turn table 12 is disposed in a treatment tank 10, and the turn table 12 is turned with a motor 16 placed outside of the treatment tank 10.

As pressure reduction means for the treatment tank 10, a vacuum pump 18 is disposed outside of the treatment tank 10, and a solenoid valve 20 is provided in a pressure reduction pipe 22 which connects the treatment tank 10 and the vacuum pump 18.

As pressure recovery means for the treatment tank 10, a pressure recovery pipe 28 having a filter 24 for filtering the drawn outside air and a solenoid valve 26 is connected to the treatment tank 10 for sucking the outside air into the treatment tank 10 under reduced pressure and recovering the pressure.

A far-infrared heater 14 is disposed in the treatment tank 10 as heating means for the amnion placed on the turn table 12 in the treatment tank 10, and a microwave irradiation device 30 is provided outside of the treatment tank 10 so that the object placed on the turn table 12 can be irradiated with microwaves. The set temperature of the heating means is a temperature at which the cell tissues constituting the object are not damaged, preferably 50°C or lower.

When viruses are inactivated using the apparatus illustrated in Fig. 6, an object to be subjected to viral inactivation is placed on the turn table 12. While the turn table 12 on which the object is placed is continuously turned with the motor 16, the object is continuously heated with the far-infrared heater 14 as the heating means.

While the object in the treatment tank 10 is continuously heated with the far-infrared heater 14, the vacuum pump 18 as the pressure reduction means is driven, and the solenoid valve 20 is opened to make the inside of the treatment tank 10 be under reduced pressure. At this point, the solenoid valve 26 as the pressure recovery means is closed.

An example of viral inactivation using the apparatus of Fig. 6 is explained.

As the microwave irradiation device 30, a magnetron with an output of 1.5 KW is used. The temperature setting of the far-infrared heater 14 is at 50°C, and the object is irradiated with far-infrared rays continuously from the start to the end. The pressure reached at the maximum pressure reduction with the vacuum pump 18 is set at 0.4 kPa when no object is placed in the treatment tank 10. The object is placed on a tray. After the tray is placed on the turn table 12 in the treatment tank 10, the turn table 12 is turned. The turn table 12 turns continuously from the start to the end.

Next, the far-infrared heater 14 is turned on, and a pressure reduction operation for reducing the pressure in the treatment tank 10 is started by driving the vacuum pump 18 and opening the solenoid valve 20. Because the rate of pressure reduction decreases a while after starting the pressure reduction, when the pressure reached at the maximum pressure reduction reaches 0.90 kPa, the vacuum pump 18 is stopped, and the solenoid valve 20 is closed. A pressure recovery operation for introducing air from which dust and bacteria have been removed by filtration through the filter 24 into the treatment tank 10 is started by opening the solenoid valve 26, and the pressure in the treatment tank 10 is recovered to 4.53 kPa.

At the same time with the start of the pressure recovery operation, the magnetron as the microwave irradiation device 30 is turned on, and the object on the turn table 12 is irradiated with microwaves.

After the heating with the far-infrared heater 14 and the microwave irradiation are carried out for three minutes, the magnetron is turned off, and the far-infrared heater 14 is turned on. At the same time, the pressure reduction operation is started again. After the pressure in the treatment tank 10 is reduced to 0.62 kPa through the restarted pressure reduction operation, a pressure recovery operation for recovering the pressure in the treatment tank 10 to 4.63 kPa, heating with the far-infrared heater 14 and microwave irradiation are carried out for three minutes. The pressure reduction operation, the heating operation and the pressure recovery operation are carried out six times in total, and the viral inactivation is completed.

The completion is determined by the pressure reached at the maximum pressure reduction in the treatment tank 10 through the fifth pressure reduction operation and the pressure reached at the maximum pressure reduction with no object placed in the treatment tank 10. That is, when the pressure reached at the maximum pressure reduction during the sixth pressure reduction operation reaches 0.40 kPa, which is equal to the pressure reached at the maximum pressure reduction with no object placed in the treatment tank 10, it is determined to complete the operations.

Regarding the viral inactivation method described above, an example in which the microwave irradiation is carried out during the pressure recovery step after the pressure in the treatment tank 10 is reduced to a predetermined pressure has been explained, but the microwave irradiation may be carried out during the pressure reduction step or carried out both during the pressure reduction step and during the pressure recovery step.

### Examples

### <Preparation of Virus Solutions and Measurement Methods of Virus Amounts by Plaque Assay>

### 1) Quantification of Herpes Simplex Virus Type 1 (HSV-1)

Vero cells (kidney-derived cells of an African green monkey) are infected with HSV-1, strain KOS and cultured in a CO₂ incubator after adding 2% fetal bovine serum-containing MEM medium. After confirming that CPE (a pathological change of the cells caused by the viral proliferation) has appeared sufficiently after 20 to 24 hours, the culture is moved to a freezer at -80°C, and the solution obtained by freezing and thawing treatment repeated three times is moved to a tube and centrifuged (3,000 rpm, 4°C, 10 minutes) to obtain a supernatant. The supernatant is dispensed into microtubes and stored at -80°C. The virus amounts were measured by the plaque assay method. That is, the virus solution was diluted with PBS (phosphate-buffered saline) by a factor of 10° to 10⁷, and Vero cells which were cultured in layers on 35-mm dishes in advance were infected using the virus solutions. Then, a medium for plaque assay (0.8% methylcellulose- and 2% fetal bovine serum-containing MEM medium) was added, and the cells were cultured in a CO₂ incubator for two days and fixed and stained with a crystal violet solution. The detected plaques were counted under a microscope. The virus amounts were measured in this manner.

### 2) Quantification of Poliovirus Type 1 (Pol-1)

Vero cells are infected with strain Sabin, which is a vaccine strain of Pol-1, and cultured in a CO₂ incubator after adding 2% fetal bovine serum-containing MEM medium. After confirming that CPE has appeared sufficiently after 20 to 24 hours, the medium is moved to a tube and centrifuged (3,000 rpm, 4°C, 10 minutes) to obtain a supernatant. The supernatant is dispensed into microtubes and stored at -80°C. The virus amounts were measured by the plaque assay method. That is, the virus solution was diluted with PBS (phosphate-buffered saline) by a factor of 10° to 10⁷, and Vero cells which were cultured in layers on 35-mm dishes in advance were infected using the virus solutions. Then, a medium for plaque assay (0.8% methylcellulose- and 2% fetal bovine serum-containing MEM medium) was added, and the cells were cultured in a CO₂ incubator for two days and fixed and stained with a crystal violet solution. The detected plaques were counted under a microscope. The virus amounts were measured in this manner.

### 3) Quantification of Influenza A Virus (IFV)

MDCK cells (canine kidney-derived cells) are infected with IFV, strain A/NWS/33 (subtype H1N1) and cultured in a CO₂ incubator after adding 2% fetal bovine serum-containing MEM medium. After confirming that CPE has appeared sufficiently after 20 to 24 hours, the medium is moved to a tube and centrifuged (3,000 rpm, 4°C, 10 minutes) to obtain a supernatant. The supernatant is dispensed into microtubes and stored at -80°C. The virus amounts were measured by the plaque assay method. That is, the virus solution was diluted with PBS (phosphate-buffered saline) by a factor of 10° to 10⁷, and MDCK cells which were cultured in layers on 35-mm dishes in advance were infected using the virus solutions. Then, a medium for plaque assay (2% agarose- and 2% fetal bovine serum-containing MEM medium) was added, and the cells were cultured in a CO₂ incubator for two days and fixed and stained with a crystal violet solution. The detected plaques were counted under a microscope. The virus amounts were measured in this manner.

### <Viral Inactivation>

The viruses in the virus solutions were inactivated using the apparatus illustrated in Fig. 6 under the following conditions for vacuum, far-infrared rays and microwaves.
- Tank heating: 50°C, F.I.R: 50°C, stop valve: 37%, maximum reached pressure of 0.34 kPa, maximum reached pressure at operation without object of 0.33 kPa
- Inactivation Method
   (1) reduce pressure 180 sec
   (2) recover pressure 30 sec (stop valve opening 37%) introduce microwaves 0.1 kw-180 sec (pressure recovery continued)
   (3) reduce pressure 180 sec
   (4) repeat (2) and (3)
   (5) check reached pressure after 180-sec pressure reduction in (3) (0.30 to 0.35 kPa), and manually complete inactivation
      complete after recovering to atmospheric pressure

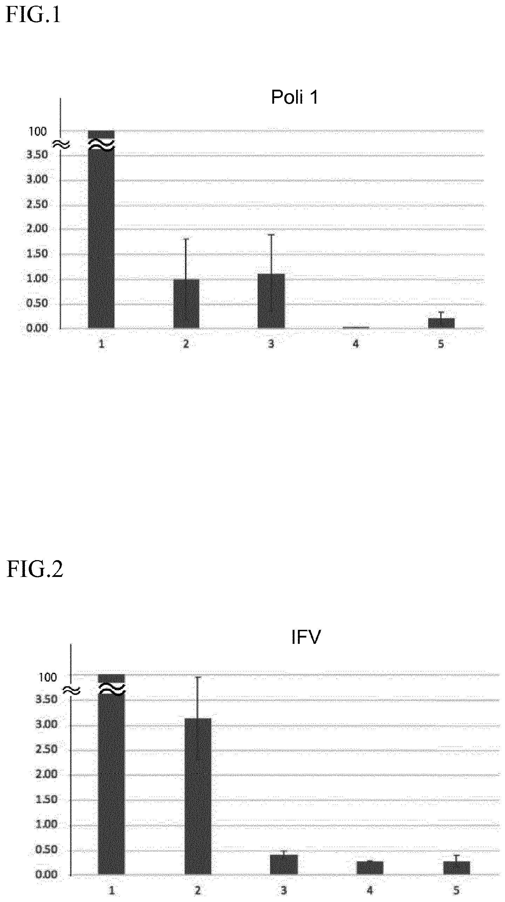

The inactivation state of the poliovirus is shown in Fig. 1.

This graph shows: 1 is without any treatment; 2 is microwaves 0 W + far-infrared rays; 3 is microwaves 100 W + far-infrared rays; 4 is microwaves 200 W + far-infrared rays; and 5 is microwaves 300 W + far-infrared rays.

It can be seen that, in the case of the poliovirus, the viral inactivation effect is high with microwaves 200 W + far-infrared rays.

The inactivation state of the influenza virus is shown in Fig. 2.

This graph shows: 1 is without any treatment; 2 is microwaves 0 W + far-infrared rays; 3 is microwaves 100 W + far-infrared rays; 4 is microwaves 200 W + far-infrared rays; and 5 is microwaves 300 W + far-infrared rays.

It can be seen that, in the case of the influenza virus, the viral inactivation effect is high with combinations of microwaves and far-infrared rays regardless of the output of the microwaves.

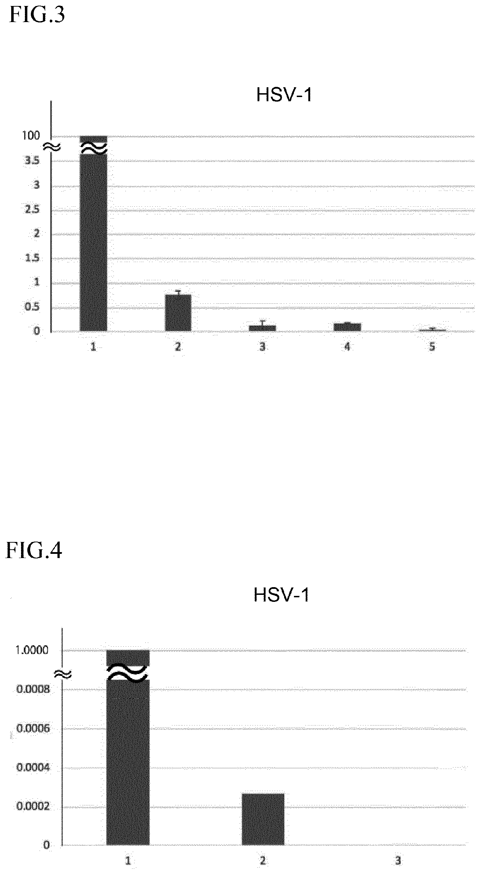

The inactivation state of herpes virus type 1 is shown in Fig. 3.

This graph shows: 1 is without any treatment; 2 is microwaves 0 W + far-infrared rays; 3 is microwaves 100 W + far-infrared rays; 4 is microwaves 200 W + far-infrared rays; and 5 is microwaves 300 W + far-infrared rays.

It can be seen that, in the case of herpes virus type 1, the viral inactivation effect is high with combinations of microwaves and far-infrared rays regardless of the output of the microwaves although far-infrared rays alone have some effect.

The infectivity of viruses decreases to several percent by pressure reduction and far-infrared rays but further decreases by microwave irradiation, and the viruses are inactivated to 0.5% or less in the end by the use of the both in combination.

That is, by controlling the three elements, namely vacuum, far-infrared rays and microwaves, the infectivity of viruses can be decreased to 1/200 to 1/1000.

The inactivation state of herpes virus type 1 without irradiation with far-infrared rays is shown in Fig. 4.

The graph shows: 1 is without any treatment; 2 is microwaves 100 W; and 3 is microwaves 100 W + far-infrared rays.

It can be seen that, when herpes virus type 1 is irradiated with microwaves under reduced pressure, viral inactivation can be achieved to some extent even without irradiation with far-infrared rays.

The inactivation state of the poliovirus without irradiation with far-infrared rays is shown in Fig. 5.

The graph shows: 1 is without any treatment; 2 is microwaves 100 W; and 3 is microwaves 100 W + far-infrared rays.

It can be seen that, when the poliovirus is irradiated with microwaves under reduced pressure, viral inactivation can be achieved to some extent even without irradiation with far-infrared rays.

Next, a method for producing a virus-inactivated dried amnion as a regenerative medicine material using the apparatus illustrated in Fig. 6 is explained.

A fresh amnion covering a fetus of an animal including human is placed on the turn table 12. As the fresh amnion, a fresh human-derived amnion, in particular, a fresh amnion aseptically separated from a placenta or the like after caesarean delivery, can be preferably used.

The fresh amnion is spread into a sheet and placed on the turn table 12 in the treatment tank 10. In particular, the fresh amnion is preferably spread into a sheet and placed on water-absorbing paper spread on the turn table 12 in the treatment tank 10. This is because the fresh amnion can be dehydrated easily.

In this manner, while the turn table 12 on which the fresh amnion is placed is continuously turned with the motor 16, the fresh amnion is continuously heated with the far-infrared heater 14 as the heating means.

While the amnion in the treatment tank 10 is continuously heated with the far-infrared heater 14, the vacuum pump 18 as the pressure reduction means is driven, and the solenoid valve 20 is opened to make the inside of the treatment tank 10 under reduced pressure. At this point, the solenoid valve 26 as the pressure recovery means is closed.

After the pressure in the treatment tank 10 reaches a predetermined pressure (for example, 0.62 kPa), the driving of the vacuum pump 18 as the pressure reduction means is stopped, and the solenoid valve 20 is closed to complete the pressure reduction step.

Then, the solenoid valve 26 as the pressure recovery means is opened, and the outside air is sucked into the treatment tank 10 to start a pressure recovery step for recovering the pressure in the treatment tank 10. The pressure recovery step is carried out in such a manner that the pressure in the treatment tank 10 becomes a predetermined pressure (for example, 4.63 kPa).

At the same time with the start of the pressure recovery step, the magnetron as the microwave irradiation device 30 is turned on, and the amnion on the turn table 12 is irradiated with microwaves. The far-infrared ray irradiation with the far-infrared heater 14 is constantly carried out during the pressure reduction step and the pressure recovery step.

During the pressure recovery step, after the heating with the far-infrared heater 14 and the microwave irradiation are carried out for a predetermined period (for example, three minutes), the magnetron is turned off, and the pressure recovery step is completed.

After the completion of the pressure recovery step, the far-infrared heater 14 is turned on, and the pressure reduction step is started again.

After the pressure in the treatment tank 10 is reduced to a predetermined pressure (for example, 0.62 kPa) through the restarted pressure reduction step, heating with the far-infrared heater 14 and microwave irradiation are carried out for three minutes during the pressure recovery step for recovering the pressure in the treatment tank 10 to a predetermined pressure (for example, 4.63 kPa).

The operations of the pressure reduction step, the heating and the pressure recovery step are repeated six times in total, and the viral inactivation is completed.

By the method described above, a virus-inactivated dried amnion which is obtained by dehydrating and drying a fresh amnion without damaging the epithelial cells, the basement membrane and the connective tissues constituting the amnion and inactivating the viruses and which retains the epithelial cells, the basement membrane and the connective tissues that are similar to those of the fresh amnion when being rehydrated by immersing in water or a buffer can be produced.

Regarding the method for producing a virus-inactivated dried amnion described above, an example in which the microwave irradiation is carried out during the pressure recovery step after the pressure in the treatment tank 10 is reduced to a predetermined pressure has been explained, but the microwave irradiation may be carried out during the pressure reduction step or carried out both during the pressure reduction step and during the pressure recovery step.

### Industrial Applicability

The viral inactivation method of the invention is useful as a viral inactivation method for drying and producing a biological material such as an amnion. Moreover, the method can also be applied to inactivation of a material for regenerative medicine itself, inactivation upon drying a material for regenerative medicine and food processing.

## Claims

1. A viral inactivation method **characterized by** inactivating a virus by microwave irradiation.

2. The viral inactivation method according to claim 1 which is **characterized by** carrying out the microwave irradiation under reduced pressure.

3. The viral inactivation method according to claim 2, wherein the reduced pressure is a low vacuum.

4. The viral inactivation method according to any one of claim 1 to claim 3, wherein the microwave has an ISM frequency.

5. The viral inactivation method according to any one of claim 1 to claim 4, wherein the object is a material for regenerative medicine.

6. A method for producing a dried amnion including simultaneously carrying out drying treatment and viral inactivation of a fresh amnion covering a fetus of an animal including human by irradiating with a microwave.

7. The method for producing a dried amnion according to claim 6 which is **characterized by** irradiating with the microwave under reduced pressure.

8. A method for producing a dried amnion
which uses a drying apparatus equipped with a treatment tank in which an amnion as the object is placed, pressure reduction means for reducing the pressure in the treatment tank, a microwave irradiation device for heating the amnion placed in the treatment tank under reduced pressure and inactivating a virus and pressure recovery means for recovering the reduced pressure in the treatment tank towards atmospheric pressure and
which includes alternately repeating a pressure reduction step of reducing the pressure in the treatment tank with the pressure reduction means and a pressure recovery step of recovering the pressure in the treatment tank with the pressure recovery means multiple times and
simultaneously carrying out drying treatment and viral inactivation by irradiating the amnion placed in the treatment tank with a microwave with the microwave irradiation device.

9. The method for producing a dried amnion according to claim 8 which is **characterized by** irradiating the amnion placed in the treatment tank with the microwave with the microwave irradiation device during the pressure recovery step with the pressure recovery means.

10. The method for producing a dried amnion according to claim 7 or claim 8 which is **characterized in that**
the drying apparatus is equipped with a far-infrared heater and
that the far-infrared heater constantly heats the amnion in the treatment tank during the pressure reduction step and the pressure recovery step which are repeated.

11. A dried amnion which is obtained through drying treatment of a fresh amnion covering a fetus of an animal including human without damaging the cell tissues of the fresh amnion and
which is **characterized by** being subjected to viral inactivation treatment during the drying treatment,
being dehydrated and dried so as to be stored in a sterilized dry atmosphere and
retaining the epithelial cells, the basement membrane and the connective tissues that are similar to those of the fresh amnion when being rehydrated by immersing in water or a buffer.
